# EUROPEAN PATENT APPLICATION

(11) **EP 1 970 063 A1**
(43) Date of publication of application: **17.09.2008**
(21) Application number: 06843523.9
(22) Date of filing: 27.12.2006
(51) Int. Cl.: A61K 31/513, A61K 31/4439, A61K 45/00, A61P 1/18, A61P 3/10

(54) **THERAPEUTIC AGENT FOR DIABETES**

(30) Priority: 28.12.2005 JP 2005379407; 07.03.2006 JP 2006061722
(71) Applicant: Takeda Pharmaceutical Company Limited, Osaka-shi, Osaka 541-0045 (JP)
(72) Inventor: TAKEUCHI, Koji, Osaka-shi Osaka 532-8686 (JP); MORITOH, Yusuke, Osaka-shi Osaka 532-8686 (JP)
(74) Representative: Dossmann, Gérard
(86) International application number: PCT/JP2006/326141
(87) International publication number: WO 2007/074884

(57) **Abstract**

The present invention relates to an agent for pancreas protection which contains a combination of a blood glucose lowering drug that does not stimulate insulin secretion and a compound represented by the formula (I) 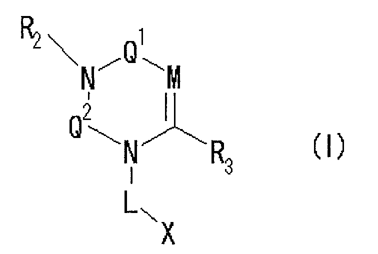 wherein each symbol is defined as in the description, or a salt thereof, or a compound represented by the formula (II) 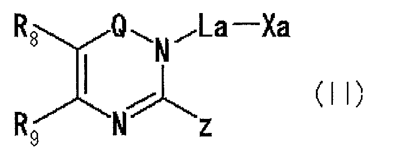 wherein each symbol is defined as in the description,
or a salt thereof.

## Description

### Technical Field

The present invention relates to an agent for pancreas protection, which is useful for the treatment of diabetes and the like.

### Background Art

It is known that the amount of pancreatic β cells remarkably decreases not only in type 1 but also type 2 diabetes (fasting blood glucose: FPG>126 mg/dL), and the decrease in the amount of pancreatic β cells has also been acknowledged in IFG (Impaired Fasting Glucose) (110<FPG<125 mg/dL) wherein the fasting blood glucose is somewhat above the normal level. While the amount of pancreatic β cells is histologically quantified as the amount of insulin-positive β cells, a pancreatic insulin content is considered to reflect the amount of pancreatic β cells and an insulin content of individual β cell. Accordingly, for the ultimate treatment of diabetes, therefore, a pharmaceutical agent positively increasing a pancreatic insulin content that reflect both the amount of pancreatic β cells and the insulin content of individual β cell is considered to be effective.
The amount of pancreatic β cells is controlled by regeneration, replication and cell death due to apoptosis of β cells and, in diabetes, pancreatic β cells are exhausted and the pancreatic β cell death is finally promoted. Accordingly, a pharmaceutical agent that increases the pancreatic insulin content and has a pancreas protection activity such as suppression of pancreatic fatigue, pancreatic β cell death, and the like is considered to be extremely effective for the treatment of diabetes.
In the present specification, compound (I) and compound (II) are known to be useful as dipeptidyl-peptidase IV (hereinafter sometimes to be abbreviated as DPP-IV) inhibitors (see US-A-2005-0261271 and EP-A-1506967).

### Disclosure of the Invention

### Problems to be Solved by the Invention

It is an object of the present invention to provide an agent for pancreas protection, which is useful for the treatment of diabetes and the like and free of side effects.

### Means of Solving the Problems

The present inventors have first found that a combination of a blood glucose lowering drug that does not stimulate insulin secretion, and compound (I) or compound (II) affords unexpectedly superior effects on increasing pancreatic insulin content, which resulted in the completion of the present invention.

Accordingly, the present invention is the following.
1) An agent for pancreas protection, which comprises a blood glucose lowering drug that does not stimulate insulin secretion, and compound (I) or compound (II) in combination (hereinafter to be sometimes abbreviated as "the agent for protecting pancreas of the present invention").
2) The agent of the aforementioned 1), wherein the blood glucose lowering drug that does not stimulate insulin secretion is an insulin sensitizer.
3) The agent of the aforementioned 2), wherein the insulin sensitizer is pioglitazone or a salt thereof.
4) The agent of the aforementioned 1), wherein the blood glucose lowering drug that does not stimulate insulin secretion is a biguanide.
5) The agent of the aforementioned 4), wherein the biguanide is metformin or a salt thereof.
6) The agent of the aforementioned 1), wherein the blood glucose lowering drug that does not stimulate insulin secretion is an α-glucosidase inhibitor.
7) The agent of the aforementioned 6), wherein the α-glucosidase inhibitor is voglibose.
8) An agent for pancreas protection, which comprises a blood glucose lowering drug that does not stimulate insulin secretion and 2-[[6-[(3R)-3-amino-1-piperidinyl]-3,4-dihydro-3-methyl-2,4-dioxo-1(2H)-pyrimidinyl]methyl]benzonitrile or a salt thereof in combination.
9) An agent for pancreas protection, which comprises a blood glucose lowering drug that does not stimulate insulin secretion and 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof in combination.
10) An agent for pancreas protection, which comprises a blood glucose lowering drug that does not stimulate insulin secretion and 2-[2-(3-(R)-aminopiperidin-1-yl)-5-fluoro-6-oxo-6H-pyrimidin-1-ylmethyl]benzonitrile or a salt thereof in combination.
11) A method of pancreas protection in a mammal, which comprises administering a blood glucose lowering drug that does not stimulate insulin secretion, and compound (I) or compound (II) to the mammal.
12) Use of a blood glucose lowering drug that does not stimulate insulin secretion, and compound (I) or compound (II) for the production of an agent for pancreas protection.

### Effect of the Invention

The agent for pancreas protection of the present invention affords superior effects in increasing pancreatic insulin content, and is useful for the treatment of diabetes and the like.
Moreover, the agent for pancreas protection of the present invention suppress pancreatic fatigue due to glucotoxicity, lipotoxicity, oxidative stress or an endoplasmic reticulum stress and the like caused by diabetes, and can maintain glucose-dependent insulin secretion activity, which is an important function of pancreatic β cells.
Moreover, the agent for pancreas protection of the present invention can suppress pancreatic β cell death due to diabetes and can promote regeneration or replication of pancreatic β cells.
Furthermore, although the agent for pancreas protection of the present invention initiates promotion of a glucose-dependent insulin secretion, it is free of side effects associated with insulin preparations (e.g., vascular complications, hypoglycemia) and blood glucose lowering drugs that stimulate insulin secretion by acting on a sulfonylurea receptor (e.g., pancreatic fatigue, hypoglycemia). Therefore, the agent for pancreas protection of the present invention can be safely administered for a long time to patients affected with diabetes and the like.

In the present specification, the blood glucose lowering drug that does not stimulate insulin secretion means a compound that lowers blood glucose by an action mechanism other than the insulin secretion from pancreatic β cells. While the compound may be peptidic or nonpeptidic, a nonpeptidic one is preferable.
In addition, the blood glucose lowering drug that does not stimulate insulin secretion may be in different forms before and after administration into the living body, as long as the blood glucose lowering activity without stimulation of insulin secretion is maintained. In other words, the blood glucose lowering drug that does not stimulate insulin secretion may be an "active metabolite" that acquires a blood glucose lowering activity without stimulation of insulin secretion after becoming a structure-modified drug by metabolism in vivo. Moreover, the blood glucose lowering drug that does not stimulate insulin secretion may be a "prodrug" that changes to an active form by reaction of enzyme, gastric acid and the like under physiological conditions in the living body.
Specific examples of the blood glucose lowering drug that does not stimulate insulin secretion, include insulin sensitizers, biguanides, somatostatin receptor agonists, 11β-hydroxysteroid dehydrogenase inhibitors, α-glucosidase inhibitors and the like. Two or more kinds thereof may be used in combination at an appropriate ratio.
Examples of the insulin sensitizer include thiazolidinedione compounds [e.g., pioglitazone or a salt thereof (preferably hydrochloride), rosiglitazone or a salt thereof (preferably maleate), Netoglitazone, Rivoglitazone, Balaglitazone, Edaglitazone], Reglixane, FK-614, the compounds described in WO01/38325, Tesaglitazar, Ragaglitazar, Muraglitazar, Naveglitazar, Metaglidasen, LY-510929, T-131 or a salt thereof, THR-0921 and the like. Of these, thiazolidinedione compounds are preferable, and further, pioglitazone and a salt thereof (preferably hydrochloride) are preferable.
Examples of the biguanides include metformin, phenformin, buformin and a salt thereof (e.g., hydrochloride, fumarate, succinate) and the like. Of these, metformin and a salt thereof (preferably hydrochloride) are preferable.
Examples of the somatostatin receptor agonists include the compounds described in WO01/25228, WO03/42204, WO98/44921, WO98/45285, WO99/22735 and the like, and the like. Of these, somatostatin subtype 2 receptor agonists are preferable.
Examples of the 11β-hydroxysteroid dehydrogenase inhibitor include BVT-3498 and the like.
Examples of the α-glucosidase inhibitors include voglibose, acarbose, miglitol, emiglitate and the like. Of these, voglibose is preferable.
The blood glucose lowering drug that does not stimulate insulin secretion is preferably an insulin sensitizer, biguanide or α-glucosidase inhibitor, more preferably an insulin sensitizer, particularly preferably pioglitazone or a salt thereof (preferably hydrochloride).

The definition of each substituent in compound (I) and compound (II) is explained in the following.
As for the "substituted or unsubstituted", the substituent includes aldehyde, alkyl, alkylene, alkylidene, amide, amino, aminoalkyl, aryl, bicycloalkyl, bicycloaryl, carbamoyl, carbocyclyl, carboxy, carbonyl group, cycloalkyl, cycloalkylene, ester, halo, heterobicycloalkyl, heterocycloalkylene, heteroaryl, heterobicycloaryl, heterocycloalkyl, oxo, hydroxy, iminoketone, ketone, nitro, oxaalkyl, oxoalkyl (each may be further substituted or unsubstituted) and the like.
The "alkyl" and (C₁₋₁₀)alkyl include C₁₋₆ alkyl such as methyl, ethyl, propyl, isopropyl, butyl, sec-butyl, isobutyl, tert-butyl and the like.
The "alkylene" includes C₁₋₆ alkylene such as methylene (-CH₂-), ethylene (-CH₂CH₂-), trimethylene (-CH₂CH₂CH₂-), tetramethylene (-CH₂CH₂CH₂CH₂-), 2-butenylene (-CH₂CH=CHCH₂-), 2-methyltetramethylene (-CH₂CH(CH₃)CH₂CH₂-), pentamethylene (-CH₂CH₂CH₂CH₂CH₂-) and the like.
Examples of the "alkylidene" include C₁₋₆ alkylidene such as methylene (=CH₂), ethylidene (=CHCH₃), isopropylidene (=C(CH₃)₂), propylidene (=CHCH₂CH₃), allylidene (=CH-CH=CH₂) and the like.
The "amino" includes -NH₂, -NHCH₃, -N(CH₃)₂, -NHC₁₋₃-alkyl, -N(C₁₋₃-alkyl)₂ and the like, and may be protected by a suitable protecting group (e.g., acetyl, tert-butoxycarbonyl, benzyloxycarbonyl etc.).
The "aryl" means a monocyclic aromatic hydrocarbon group, and (C₆₋₁₀)aryl such as phenyl and the like.
The "bicycloaryl" means a bicyclic aromatic hydrocarbon group, and (C₉₋₁₂)bicycloaryl such as naphthyl and the like.
The "carbocyclyl" means a ring consisting of carbon atoms.
The "cycloalkyl" and (C₃₋₁₂)cycloalkyl include cyclopropyl, cyclobutyl, cyclopentyl, cyclohexyl, cyclohexenyl, 2,5-cyclohexadienyl, bicyclo[2.2.2]octyl, adamant-1-yl, decahydronaphthyl, oxocyclohexyl, dioxocyclohexyl, thiocyclohexyl, 2-oxobicyclo[2.2.1]hept-1-yl and the like.
The "bicycloalkyl" includes bicyclo[2.2.2]octyl, decahydronaphthyl, 2-oxobicyclo[2.2.1]hept-1-yl and the like.
The "cycloalkylene" includes a divalent group derived from the aforementioned cycloalkyl.
The "halo" means fluoro, chloro, bromo or iodo.
The "heterobicycloalkyl" includes 3-azabicyclo[4.1.0]hept-3-yl, 2-azabicyclo[3.1.0]hex-2-yl, 3-azabicyclo[3.1.0]hex-3-yl and the like.
The "heterocycloalkylene" includes a divalent group derived from the aforementioned heterocycloalkyl.
The "heteroaryl" means a cyclic aromatic group containing 5 or 6 ring constituting atoms, wherein at least one of the atoms is a hetero atom (e.g., nitrogen atom, oxygen atom, sulfur atom) and the rest are carbon atoms. Here, the nitrogen atom may be quaternized, and the sulfur atom may be oxidized. The heteroaryl includes a group derived from furan, imidazole, isothiazole, isoxazole, oxadiazol, oxazole, 1,2,3-oxadiazol, pyrazine, pyrazole, pyridazine, pyridine, pyrimidine, pyrroline, thiazole, 1,3,4-thiadiazol, triazole or tetrazole.
The "heteroaryl" includes a bicyclic or tricyclic ring wherein the heteroaryl ring is independently condensed with 1 or 2 rings selected from the group consisting of aryl ring, cycloalkyl ring, cycloalkenyl ring and other monocyclic heteroaryl and heterocycloalkyl ring. The bicyclic or tricyclic heteroaryl includes a group derived from benzo[b]furan, benzo[b]thiophene, benzimidazole, imidazo[4,5-c]pyridine, quinazoline, thieno[2,3-c]pyridine, thieno[3,2-b]pyridine, thieno[2,3-b]pyridine, indolizine, imidazo[1,2-a]pyridine, quinoline, isoquinoline, phthalazine, quinoxaline, naphthyridine, quinolizidine, indole, isoindole, indazole, indoline, benzoxazole, benzopyrazole, benzothiazole, imidazo[1,5-a]pyridine, pyrazolo[1,5-a]pyridine, imidazo[1,2-a]pyrimidine, imidazo[1,2-c]pyrimidine, imidazo[1,5-a] pyrimidine, imidazo[1,5-c] pyrimidine, pyrrolo[2,3-b]pyridine, pyrrolo[2,3-c]pyridine, pyrrolo[3,2-b]pyridine, pyrrolo[3,2-c]pyridine, pyrrolo[2,3-d]pyrimidine, pyrrolo[3,2-d]pyrimidine, pyrrolo[2,3-b]pyrazine, pyrazolo[1,5-a]pyridine, pyrrolo[1,2-b]pyridazine, pyrrolo[1,2-c]pyrimidine, pyrrolo[1,2-a]pyrimidine, pyrrolo[1,2-a]pyrazine, triazo[1,5-a]pyridine, pteridin, purine, carbazole, acridine, phenazine, phenothiazene, phenoxathiine, 1,2-dihydropyrrolo[3,2,1-hi]indole, indolizine, pyrido[1,2-a]indole and 2(1H)-pyridinone. The bicyclic or tricyclic ring may be bonded to its parent molecule via heteroaryl itself, or aryl, cycloalkyl, cycloalkenyl or heterocycloalkyl, which is condensed with the heteroaryl.
The "heterobicycloaryl" and hetero(C₄₋₁₀)bicycloaryl include 2-amino-4-oxo-3,4-dihydropteridin-6-yl, tetrahydroisoquinolinyl and the like.
The "heterocycloalkyl" and hetero(C₃₋₁₂)cycloalkyl include piperidyl, 4-morpholyl, 4-piperazinyl, pyrrolidinyl, perhydropyrrolidinyl, 1,4-diazaperhydroepinyl, 1,3-dioxanyl, 1,4-dioxanyl and the like.
The (C₆₋₁₀)aryl(C₁₋₁₀)alkyl includes benzyl, phenethyl, 1-phenylethyl, 3-phenylpropyl and the like.

In the present specification, compound (I) is a compound represented by the formula:

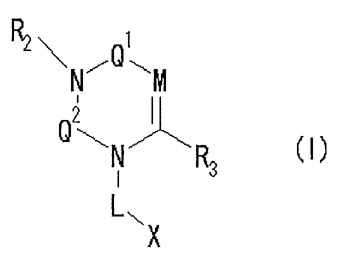

wherein M is N or CR₄;
Q¹ and Q² are each independently selected from the group consisting of CO, CS, SO, SO₂ and C=NR₁;
R₂ is a hydrogen atom, or a group selected from the group consisting of (C₁₋₁₀)alkyl, (C₃₋₁₂)cycloalkyl, (C₃₋₁₂)cycloalkyl(C₁₋₅)alkyl, hetero(C₃₋₁₂)cycloalkyl(C₁₋₅)alkyl, hetero(C₃₋₁₂)cycloalkyl, (C₆₋₁₀)aryl(C₁₋₁₀)alkyl, heteroaryl(C₁₋₅)alkyl, (C₉₋₁₂)bicycloaryl, hetero(C₄₋₁₂)bicycloaryl, hetero(C₄₋₁₂)bicycloaryl(C₁₋₅)alkyl, carbonyl(C₁₋₃)alkyl, thiocarbonyl(C₁₋₃)alkyl, sulfonyl(C₁₋₃)alkyl, sulfinyl(C₁₋₃)alkyl, imino (C₁₋₃)alkyl, amino, (C₆₋₁₀)aryl, heteroaryl, hydroxy, (C₁₋₁₀)alkoxy, (C₆₋₁₀)aryloxy, heteroaryloxy, carbonyl group, imino group, sulfonyl group and sulfinyl group (each of these can be substituted or unsubstituted);
R₃ is a group selected from the group consisting of perhalo(C₁₋₁₀)alkyl, amino, (C₁₋₁₀)alkyl, (C₃₋₁₂)cycloalkyl, hetero(C₃₋₁₂)cycloalkyl, (C₆₋₁₀)aryl(C₁₋₁₀)alkyl, heteroaryl(C₁₋₅)alkyl, (C₆₋₁₀)aryl, heteroaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, hydroxy, (C₁₋₁₀)alkoxy, (C₆₋₁₀)aryloxy, heteroaryloxy, carbonyl group, imino group, sulfonyl group and sulfinyl group (each of these can be substituted or unsubstituted), and a substituted or unsubstituted 3-, 4-, 5-, 6- or 7-membered ring;
R₄ is a hydrogen atom, or a group selected from the group consisting of halo, perhalo(C₁₋₁₀)alkyl, amino, cyano, thio, (C₁₋₁₀)alkyl, (C₃₋₁₂)cycloalkyl, hetero(C₃₋₁₂)cycloalkyl, (C₆₋₁₀)aryl(C₁₋₁₀)alkyl, heteroaryl(C₁₋₅)alkyl, (C₆₋₁₀)aryl, heteroaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, hydroxy, (C₁₋₁₀)alkoxy, (C₆₋₁₀)aryloxy, heteroaryloxy, a carbonyl group, an imino group, a sulfonyl group and a sulfinyl group (each of these can be substituted or unsubstituted);
R₁ is a hydrogen atom, or a group selected from the group consisting of (C₁₋₁₀)alkyl, (C₃₋₁₂)cycloalkyl, hetero(C₃₋₁₂)cycloalkyl, (C₆₋₁₀)aryl(C₁₋₁₀)alkyl, heteroaryl(C₁₋₅)alkyl, (C₉₋₁₂)bicycloaryl, and hetero(C₄₋₁₂)bicycloaryl (each of these can be substituted or unsubstituted);
L is a linker providing a gap (separation) corresponding to 1, 2 or 3 atoms between X and the ring L is bonded to (the atom of the linker is selected from the group consisting of a carbon atom, an oxygen atom, a nitrogen atom and a sulfur atom); and
X is a group selected from the group consisting of (C₁₋₁₀)alkyl, (C₃₋₁₂)cycloalkyl, hetero(C₃₋₁₂)cycloalkyl, (C₆₋₁₀)aryl(C₁₋₁₀)alkyl, heteroaryl(C₁₋₅)alkyl, (C₉₋₁₂)bicycloaryl, hetero(C₄₋₁₂)bicycloaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, amino, (C₆₋₁₀)aryl, heteroaryl, hydroxy, (C₁₋₁₀)alkoxy, (C₆₋₁₀)aryloxy, heteroaryloxy, (C₂₋₆)alkenyl, (C₂₋₆)alkynyl, a carbonyl group, cyano, an imino group, a sulfonyl group and a sulfinyl group (each of these can be substituted or unsubstituted), or a salt thereof.

Here, M is preferably CR₄, more preferably CH.
Q¹ and Q² are preferably CO.
R₂ is preferably a (C₁₋₄)alkyl group, more preferably methyl.
R₃ is preferably a substituted or unsubstituted 4-, 5-, 6- or 7-membered heterocycloalkyl, more preferably a group represented by the formula:

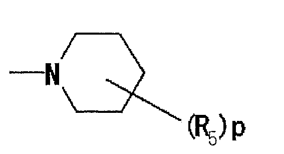

wherein p is 0-10; R₅ is each independently halo, perhalo(C₁₋₁₀)alkyl, CF₃, cyano, nitro, hydroxy, (C₁₋₁₀)alkyl, (C₆₋₁₀)aryl, heteroaryl, aminosulfonyl, (C₁₋₃)alkylsulfonyl, (C₆₋₁₀)arylsulfonyl, heteroarylsulfonyl, (C₆₋₁₀)aryloxy, heteroaryloxy, (C₆₋₁₀)aryl(C₁₋₁₀)alkyl, heteroaryl(C₁₋₅)alkyl, (C₃₋₁₂)cycloalkyl, hetero(C₃₋₁₂)cycloalkyl, amino, thio, (C₁₋₁₀)alkoxy, a carbonyl group, an imino group, a sulfonyl group or a sulfinyl group (each of these can be substituted or unsubstituted). R₃ is particularly preferably 3-aminopiperidin-1-yl.
R₄ is preferably a hydrogen atom.
The linker for L is preferably -CH₂-, -CH₂CH₂-, - CH₂CH₂CH₂-, -C(O)-, -CH₂C(O)-, -C(O)CH₂-, -CH₂C(O)CH₂-, C(O)CH₂CH₂-, -CH₂CH₂C(O)-, -O-, -OCH₂-, -CH₂O-, -CH₂OCH₂-, - OCH₂CH₂-, -CH₂CH₂O- -N(CH₃)-, -NHCH₂-, -CH₂NH-, -CH₂NHCH₂-, - NHCH₂CH₂-, -CH₂CH₂NH-, -NHC(O)-, -N(CH₃)C(O)-, -C(O)NH-, - C(O)N(CH₃)-, -NHC(O)CH₂-_{,} -C(O)NHCH₂-, -C(O)CH₂NH-, -CH₂NHC(O)-, -CH₂C(O)NH-, -NHCH₂C(O)-, -S-, -SCH₂-, -CH₂S-, -SCH₂CH₂-, - CH₂SCH₂-, -CH₂CH₂S-, -C(O)S-, -C(O)SCH₂-, -CH₂C(O)S-, -C(O)CH₂S-, -CH₂SC(O)- and the like, each of which can be substituted or unsubstituted. L is particularly preferably -CH₂-.

X is preferably a group represented by the formula:

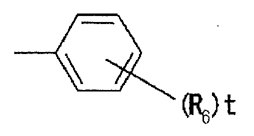

wherein t is 0, 1, 2, 3, 4 or 5; R₆ is each independently halo, perhalo(C₁₋₁₀)alkyl, CF₃, (C₁₋₁₀)alkyl, (C₂₋₆)alkenyl, (C₂₋₆)alkynyl, (C₆₋₁₀)aryl, heteroaryl, aminosulfonyl, (C₁₋₃)alkylsulfonyl, (C_{6- 10})arylsulfonyl, hetero(C₆₋₁₀)arylsulfonyl, (C₆₋₁₀)aryloxy, heteroaryloxy, (C₆₋₁₀)aryl(C₁₋₁₀)alkyl, heteroaryl(C₁₋₅)alkyl, (C₃₋₁₂)cycloalkyl, hetero(C₃₋₁₂)cycloalkyl, amino, thio, cyano, nitro, hydroxy, (C₁₋₁₀)alkoxy, a carbonyl group, an imino group, a sulfonyl group or a sulfinyl group (each of these can be substituted or unsubstituted).
X is more preferably (2-cyano)phenyl, (3-cyano)phenyl, (2-hydroxy)phenyl, (3-hydroxy)phenyl, (2-(C₂₋₆)alkenyl)phenyl, (3-(C₂₋₆)alkenyl)phenyl, (2-(C₂₋₆)alkynyl)phenyl, (3-(C₂₋₆)alkynyl)phenyl, (2-methoxy)phenyl, (3-methoxy)phenyl, (2-nitro)phenyl, (3-nitro)phenyl, (2-carboxy)phenyl, (3-carboxy)phenyl, (2-carboxamide)phenyl, (3-carboxamide)phenyl, (2-sulfonamido)phenyl, (3-sulfoamido)phenyl, (2-tetrazolyl)phenyl, (3-tetrazolyl)phenyl, (2-aminomethyl)phenyl, (3-aminomethyl)phenyl, (2-hydroxymethyl)phenyl, (3-hydroxymethyl)phenyl, (2-phenyl)phenyl, (3-phenyl)phenyl, (2-halo)phenyl, (3-halo)phenyl, (2-CONH₂)phenyl, (3-CONH₂)phenyl, (2-CONH(C₁₋₇)alkyl)phenyl, (3-CONH(C₁₋₇)alkyl)phenyl, (2-CO₂(C₁₋₇)alkyl)phenyl, (3-CO₂(C₁₋₇)alkyl)phenyl, (2-NH₂)phenyl, (3-NH₂)phenyl, (2-(C₃₋₇)alkyl)phenyl, (3-(C₃₋₇)alkyl)phenyl, (2-(C₃₋₇)cycloalkyl)phenyl, (3-(C₃₋₇)cycloalkyl) phenyl, (2-(C₆₋₁₀)aryl)phenyl, (3-(C₆₋₁₀)aryl)phenyl, (2-heteroaryl)phenyl, (3-heteroaryl)phenyl, 2-bromo-5-fluorophenyl, 2-chloro-5-fluorophenyl, 2-cyano-5-fluorophenyl, 2,5-dichlorophenyl, 2,5-difluorophenyl, 2,5-dibromophenyl, 2-bromo-3,5-difluorophenyl, 2-chloro-3,5-difluorophenyl, 2,3,5-trifluorophenyl, 2,3,5,6-tetrafluorophenyl, 2-bromo-3,5,6-trifluorophenyl, 2-chloro-3,5,6-trifluorophenyl, 2-cyano-3,5-difluorophenyl, 2-cyano-3,5,6-trifluorophenyl, (2-hetero(C₃₋₁₂)cycloalkyl)phenyl, (3-hetero(C₃₋₁₂)cycloalkyl)phenyl and the like, each of which can be substituted or unsubstituted.
X is particularly preferably (2-cyano)phenyl or 2-cyano-5-fluorophenyl.

When compound (I) forms a salt, the salt is preferably a pharmacologically acceptable salt, such as a salt with an inorganic acid, a salt with an organic acid, a salt with a basic or acidic amino acid and the like.
Preferable examples of the salt with an inorganic acid include salts with hydrochloric acid, hydrobromic acid, nitric acid, sulfuric acid, phosphoric acid and the like.
Preferable examples of the salt with an organic acid include salts with acetic acid, trifluoroacetic acid, fumaric acid, oxalic acid, tartaric acid, maleic acid, citric acid, succinic acid, malic acid, methanesulfonic acid, benzoic acid, toluenesulfonate and the like.
Preferable examples of the salt with a basic amino acid include salt with arginine and the like.
Preferable examples of the salts with acidic amino acid include salts with aspartic acid, glutamic acid and the like.
Compound (I) can be produced according to, for example, the methods described in US-A-2005-0261271, EP-A-1506967, or a method analogous thereto.

Preferable specific examples of compound (I) are the following compounds and salts thereof.
2-(6-chloro-2,4-dioxo-3,4-dihydro-2H-pyrimidin-1-ylmethyl)benzonitrile;
2-(6-chloro-3-methyl-2,4-dioxo-3,4-dihydro-2H-pyrimidin-1-ylmethyl)benzonitrile;
2-[[6-[3-amino-1-piperidinyl]-3,4-dihydro-3-methyl-2,4-dioxo-1(2H)-pyrimidinyl]methyl]benzonitrile;
2-[[6-[(3R)-3-amino-1-piperidinyl]-3,4-dihydro-3-methyl-2,4-dioxo-1(2H)-pyrimidinyl]methyl]benzonitrile;
2-[6-(3-aminopiperidin-1-yl)-3-ethyl-2,4-dioxo-3,4-dihydro-2H-pyrimidin-1-ylmethyl]benzonitrile;
2-[6-(3-aminopiperidin-1-yl)-2,4-dioxo-3,4-dihydro-2H-pyrimidin-1-ylmethyl]benzonitrile;
2-[6-(3-aminopiperidin-1-yl)-5-chloro-3-methyl-2,4-dioxo-3,4-dihydro-2H-pyrimidin-1-ylmethyl]benzonitrile;
6-(3-aminopiperidin-1-yl)-1-(2-bromobenzyl)-1H-pyrimidine-2,4-dione;
6-(3-aminopiperidin-1-yl)-1-(2-iodobenzyl)-1H-pyrimidine-2,4-dione;
6-(3-aminopiperidin-1-yl)-1-(2-bromo-5-fluorobenzyl)-3-methyl-1H-pyrimidine-2,4-dione;
6-(3-aminopiperidin-1-yl)-1-(2-chloro-5-fluorobenzyl)-3-methyl-1H-pyrimidine-2,4-dione;
6-(3-aminopiperidin-1-yl)-1-(2-chloro-4-fluorobenzyl)-3-methyl-1H-pyrimidine-2,4-dione;
6-(3-aminopiperidin-1-yl)-1-(2-bromobenzyl)-3-methyl-1H-pyrimidine-2,4-dione;
2-{6-[azepan-3(±)-ylamino]-3-methyl-2,4-dioxo-3,4-dihydro-2H-pyrimidin-1-ylmethyl}benzonitrile;
2-{6-[3(±)-aminoazepan-1-yl]-3-methyl-2,4-dioxo-3,4-dihydro-2H-pyrimidin-1-ylmethyl}benzonitrile;
2-[6-(2-aminoethylamino)-3-ethyl-2,4-dioxo-3,4-dihydro-2H-pyrimidin-1-ylmethyl]benzonitrile;
2-[6-(3-aminopiperidin-1-yl)-3-(3-cyanobenzyl)-2,4-dioxo-3,4-dihydro-2H-pyrimidin-1-ylmethyl]benzonitrile;
2-[6-(3-aminopiperidin-1-yl)-3-(2-cyanobenzyl)-2,4-dioxo-3,4-dihydro-2H-pyrimidin-1-ylmethyl]benzonitrile;
2-[6-(3-aminopiperidin-1-yl)-3-(4-cyanobenzyl)-2,4-dioxo-3,4-dihydro-2H-pyrimidin-1-ylmethyl]benzonitrile;
2-[6-(3-aminopiperidin-1-yl)-3-(1H-benzimidazol-2-ylmethyl)-2,4-dioxo-3,4-dihydro-2H-pyrimidin-1-ylmethyl]benzonitrile;
2-[6-(3-aminopiperidin-1-yl)-2,4-dioxo-3-(4-pyrazol-1-ylbenzyl)-3,4-dihydro-2H-pyrimidin-1-ylmethyl]benzonitrile;
2-[6-(3-aminopiperidin-1-yl)-2,4-dioxo-3-(3-pyrrol-1-ylbenzyl)-3,4-dihydro-2H-pyrimidin-1-ylmethyl]benzonitrile;
6-[(3-aminopiperidin-1-yl)-3-(2-cyanobenzyl)-2,6-dioxo-3,6-dihydro-2H-pyrimidin-1-ylmethyl]-thiophene-3-carbonitrile;
3-[4-(3-aminopiperidin-1-yl)-3-(2-cyanobenzyl)-2,6-dioxo-3,6-dihydro-2H-pyrimidin-1-ylmethyl]-benzoic acid methyl ester;
3-[4-(3-aminopiperidin-1-yl)-3-(2-cyanobenzyl)-2,6-dioxo-3,6-dihydro-2H-pyrimidin-1-ylmethyl]-benzoic acid;
6-(3-aminopiperidin-1-yl)-1,3-bis-(2-bromo-5-fluorobenzyl)-1H-pyrimidine-2,4-dione;
2-{6-[3(R)-aminopiperidin-1-yl]-5-chloro-2,4-dioxo-3,4-dihydro-2H-pyrimidin-1-ylmethyl}benzonitrile;
6-[3(R)-aminopiperidin-1-yl]-1-(2,5-dichlorobenzyl)-3-methyl-1H-pyrimidine-2,4-dione;
6-[3(R)-aminopiperidin-1-yl]-1-(2-chloro-3,6-difluorobenzyl)-3-methyl-1H-pyrimidine-2,4-dione;
(R)-2-((6-(3-amino-3-methylpiperidin-1-yl)-3-methyl-2,4-dioxo-3,4-dihydropyrimidin-1(2H)-yl)methyl)-4-fluorobenzonitrile;
2-[[6-[3-amino-1-piperidinyl]-3,4-dihydro-3-methyl-2,4-dioxo-1(2H)-pyrimidinyl]methyl]-4-fluorobenzonitrile; and
2-[[6-[(3R)-3-amino-1-piperidinyl]-3,4-dihydro-3-methyl-2,4-dioxo-1(2H)-pyrimidinyl]methyl]-4-fluorobenzonitrile.

Compound (I) is preferably
2-[[6-[3-amino-1-piperidinyl]-3,4-dihydro-3-methyl-2,4-dioxo-1(2H)-pyrimidinyl]methyl]benzonitrile;
2-[[6-[(3R)-3-amino-1-piperidinyl]-3,4-dihydro-3-methyl-2,4-dioxo-1(2H)-pyrimidinyl]methyl]benzonitrile;
2-[[6-[3-amino-1-piperidinyl]-3,4-dihydro-3-methyl-2,4-dioxo-1(2H)-pyrimidinyl]methyl]-4-fluorobenzonitrile;
2-[[6-[(3R)-3-amino-1-piperidinyl]-3,4-dihydro-3-methyl-2,4-dioxo-1(2H)-pyrimidinyl]methyl]-4-fluorobenzonitrile; or
a salt thereof (preferably benzoate, succinate, trifluoroacetate, tartrate, toluenesulfonate or hydrochloride).
Compound (I) is more preferably 2-[[6-[(3R)-3-amino-1-piperidinyl]-3,4-dihydro-3-methyl-2,4-dioxo-1(2H)-pyrimidinyl]methyl]benzonitrile or a salt thereof (preferably, trifluoroacetate, benzoate, hydrochloride or toluenesulfonate), particularly preferably 2-[[6-[(3R)-3-amino-1-piperidinyl]-3,4-dihydro-3-methyl-2,4-dioxo-1(2H)-pyrimidinyl]methyl]benzonitrile benzoate (to be sometimes abbreviated as compound A in the present specification). In the present specification, the free form of compound A means 2-[[6-[(3R)-3-amino-1-piperidinyl]-3,4-dihydro-3-methyl-2,4-dioxo-1(2H)-pyrimidinyl]methyl]benzonitrile.
Alternatively, compound (I) is more preferably 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof (preferably, trifluoroacetate, succinate or hydrochloride), particularly preferably 2-[[6-[(3R)-3-amino-1-piperidinyl]-3,4-dihydro-3-methyl-2,4-dioxo-1(2H)-pyrimidinyl]methyl]-4-fluorobenzonitrile succinate (to be sometimes abbreviated as compound B in the present specification). In the present specification, the free form of compound B means 2-[[6-[(3R)-3-amino-1-piperidinyl]-3,4-dihydro-3-methyl-2,4-dioxo-1(2H)-pyrimidinyl]methyl]-4-fluorobenzonitrile.

In the present specification, compound (II) is a compound represented by the formula:

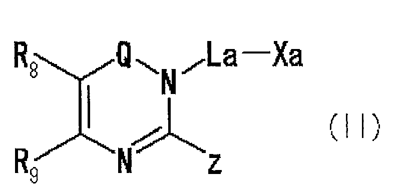

wherein Q is selected from the group consisting of CO, SO, SO₂ and C=NR₇;
Z is a group selected from the group consisting of halo, perhalo(C₁₋₁₀)alkyl, amino, cyano, thio, (C₁₋₁₀)alkyl, cycloalkyl, heterocycloalkyl, aryl, heteroaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, hydroxy, alkoxy, aryloxy, heteroaryloxy, alkenyl, alkynyl, a carbonyl group, an imino group, a sulfonyl group and a sulfinyl group (each of these can be substituted or unsubstituted), and a substituted or unsubstituted 4-, 5-, 6- or 7-membered ring;
R₈ and R₉ are each independently a hydrogen atom, or a group selected from the group consisting of halo, perhalo(C₁₋₁₀)alkyl, amino, cyano, nitro, thio, (C₁₋₁₀)alkyl, (C₃₋₁₂)cycloalkyl, hetero(C₃₋₁₂) cycloalkyl, aryl(C₁₋₁₀)alkyl, heteroaryl(C₁₋₅)alkyl, (C₉₋₁₂)bicycloaryl, hetero(C₈₋₁₂)bicycloaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, aryl, heteroaryl, hydroxy, alkoxy, aryloxy, heteroaryloxy, alkenyl, alkynyl, a carbonyl group, an imino group, a sulfonyl group and a sulfinyl group (each of these can be substituted or unsubstituted);
R₇ is a hydrogen atom, or a group selected from the group consisting of (C₁₋₁₀)alkyl, cycloalkyl, heterocycloalkyl, aryl, heteroaryl, arylalkyl, heteroarylalkyl, bicycloaryl and heterobicycloaryl (each of these can be substituted or unsubstituted);
La is a linker providing a gap (separation) corresponding to 0 to 6 atoms between Xa and the ring La is bonded to;
Xa is a group selected from the group consisting of (C₁₋₁₀)alkyl, (C₃₋₁₂)cycloalkyl, hetero(C₃₋₁₂)cycloalkyl, aryl(C₁₋₁₀)alkyl, heteroaryl(C₁₋₅)alkyl, (C₉₋₁₂)bicycloaryl, hetero(C₄₋₁₂)bicycloaryl, carbonyl (C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, amino, aryl, heteroaryl, hydroxy, alkoxy, aryloxy, heteroaryloxy, alkenyl, alkynyl, a carbonyl group, cyano, an imino group, a sulfonyl group and a sulfinyl group (each of these can be substituted or unsubstituted), or a salt thereof.

Here, Q is preferably CO.
Z is preferably a substituted or unsubstituted 4-, 5-, 6- or 7-membered ring, more preferably a group represented by the formula:

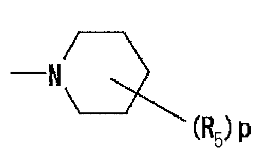

wherein p and R₅ are as defined above. Z is particularly preferably 3-amino-piperidinyl-1-yl.
R₈ is preferably halo, more preferably fluoro.
R₉ is preferably a hydrogen atom.
The linker for La is preferably -CH₂-, -CH₂CH₂-, - CH₂CH₂CH₂-, -C(O)-, -CH₂C(O)-, -C(O)CH₂-, -CH₂C(O)CH₂-, - C(O)CH₂CH₂-, -CH₂CH₂C(O)-, -O-, -OCH₂-, -CH₂O-, -CH₂OCH₂-, - OCH₂CH₂-, -CH₂CH₂O-, -N(CH₃)-, -NHCH₂-, -CH₂NH-, -CH₂NHCH₂-, - NHCH₂CH₂-, -CH₂CH₂NH-, -NHC(O)-, -N(CH₃)C(O)-, -C(O)NH-, - C(O)N(CH₃)-, -NHC(O)CH₂-, -C(O)NHCH₂-, -C(O)CH₂NH-, -CH₂NHC(O)-, -CH₂C(O)NH-, -NHCH₂C(O)-, -S-, -SCH₂-, -CH₂S-, -SCH₂CH₂-, - CH₂SCH₂-, -CH₂CH₂S-, -C(O)S-, -C(O)SCH₂-, -CH₂C(O)S-, -C(O)CH₂S-, -CH₂SC(O)- and the like. La is particularly preferably -CH₂-.

Xa is preferably a group represented by the formula:

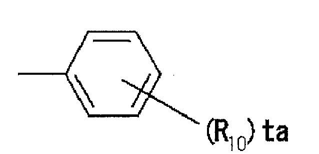

wherein ta is 0, 1, 2 or 3; each R₁₀ is independently halo, perhalo(C₁₋₁₀)alkyl, CF₃, (C₁₋₁₀) alkyl, (C₂₋₆)alkenyl, (C₂₋₆)alkynyl, (C₆₋₁₀) aryl, heteroaryl, aminosulfonyl, (C₁₋₃)alkylsulfonyl, (C₆₋₁₀)arylsulfonyl, hetero(C₆₋₁₀)arylsulfonyl, (C₆₋₁₀)aryloxy, heteroaryloxy, (C₆₋₁₀)aryl(C₁₋₁₀)alkyl, heteroaryl (C₁₋₅)alkyl, (C₃₋₁₂)cycloalkyl, hetero(C₃₋₁₂)cycloalkyl, amino, thio, cyano, nitro, hydroxy, (C₁₋₁₀)alkoxy, a carbonyl group, an imino group, a sulfonyl group or a sulfinyl group (each of these can be substituted or unsubstituted).
Xa is more preferably (2-cyano)phenyl, (3-cyano)phenyl, (2-hydroxy)phenyl, (3-hydroxy)phenyl, (2-(C₂₋₆)alkenyl)phenyl, (3-(C₂₋₆)alkenyl)phenyl, (2-(C₂₋₆)alkynyl)phenyl, (3-(C₂₋₆)alkynyl)phenyl, (2-nitro)phenyl, (3-nitro)phenyl, (2-carboxy)phenyl, (3-carboxy)phenyl, (2-carboxamide)phenyl, (3-carboxamide)phenyl, (2-sulfoneamide)phenyl, (3-sulfoneamide)phenyl, (2-tetrazolyl)phenyl, (3-tetrazolyl)phenyl, (2-aminomethyl)phenyl, (3-aminomethyl)phenyl, (2-amino)phenyl, (3-amino)phenyl, (2-hydroxymethyl)phenyl, (3-hydroxymethyl)phenyl, (2-phenyl)phenyl, (3-phenyl)phenyl, (2-CONH₂)phenyl, (3-CONH₂)phenyl, (2-CONH(C₁₋₇)alkyl)phenyl, (3-CONH(C₁₋₇)alkyl)phenyl, (2-CO₂(C₁₋₇)alkyl)phenyl, (3-CO₂(C₁₋₇)alkyl)phenyl and the like, each of which can be substituted or unsubstituted.
Xa is particularly preferably (2-cyano)phenyl.

When compound (II) forms a salt, the salt is preferably a pharmacologically acceptable salt similar to compound (I).
Compound (II) can be produced according to, for example, the methods described in US-A-2005-0261271 and EP-A-1506967, or a method analogous thereto.

Preferable specific examples of compound (II) include the following compounds and salts thereof.
2-[2-(3-(R)-aminopiperidin-1-yl)-5-bromo-6-oxo-6H-pyrimidin-1-ylmethyl]benzonitrile;
2-[2-(3-(R)-aminopiperidin-1-yl)-5-ethynyl-6-oxo-6H-pyrimidin-1-ylmethyl]benzonitrile;
2-[2-(3-(R)-aminopiperidin-1-yl)-4,5-dimethyl-6-oxo-6H-pyrimidin-1-ylmethyl]benzonitrile;
2-[2-(3-(R)-aminopiperidin-1-yl)-5-chloro-6-oxo-6H-pyrimidin-1-ylmethyl]benzonitrile;
(R)-2-((2-(3-aminopiperidin-1-yl)-5-fluoro-6-oxopyrimidin-1(6H)-yl)methyl)-4-fluorobenzonitrile;
(R)-2-((2-(3-aminopiperidin-1-yl)-5-chloro-6-oxopyrimidin-1(6H)-yl)methyl)-4-fluorobenzonitrile;
(R)-2-((2-(3-aminopiperidin-1-yl)-5-bromo-6-oxopyrimidin-1(6H)-yl)methyl)-4-fluorobenzonitrile;
2-[2-(3-(R)-aminopiperidin-1-yl)-5-prop-1-ynyl-6-oxo-6H-pyrimidin-1-ylmethyl]benzonitrile;
2-[2-(3-aminopiperidin-1-yl)-5-fluoro-6-oxo-6H-pyrimidin-1-ylmethyl]benzonitrile;
2-[2-(3-(R)-aminopiperidin-1-yl)-5-fluoro-6-oxo-6H-pyrimidin-1-ylmethyl]benzonitrile; and
2-[2-(3-(R)-amino-3-methylpiperidin-1-yl)-5-chloro-6-oxo-6H-pyrimidin-1-ylmethyl]benzonitrile.

Compound (II) is preferably
2-[2-(3-aminopiperidin-1-yl)-5-fluoro-6-oxo-6H-pyrimidin-1-ylmethyl]benzonitrile;
2-[2-(3-(R)-aminopiperidin-1-yl)-5-fluoro-6-oxo-6H-pyrimidin-1-ylmethyl]benzonitrile;
or a salt thereof (preferably trifluoroacetate, tartrate or hydrochloride).

Compound (II) is more preferably 2-[2-(3-(R)-aminopiperidin-1-yl)-5-fluoro-6-oxo-6H-pyrimidin-1-ylmethyl]benzonitrile or a salt thereof (preferably trifluoroacetate, tartrate or hydrochloride), particularly preferably, 2-[2-(3-(R)-aminopiperidin-1-yl)-5-fluoro-6-oxo-6H-pyrimidin-1-ylmethyl]benzonitrile tartrate (to be sometimes abbreviated as compound C in the present specification). In the present specification, the free form of compound C means 2-[2-(3-(R)-aminopiperidin-1-yl)-5-fluoro-6-oxo-6H-pyrimidin-1-ylmethyl]benzonitrile.

Preferable specific examples of the agent for pancreas protection of the present invention include are as follows:
(1) an agent for pancreas protection, which comprises a blood glucose lowering drug that does not stimulate insulin secretion and 2-[[6-[(3R)-3-amino-1-piperidinyl]-3,4-dihydro-3-methyl-2,4-dioxo-1(2H)-pyrimidinyl]methyl]benzonitrile or a salt thereof in combination
   [here, the blood glucose lowering drug that does not stimulate insulin secretion is preferably the following:
   (1a) insulin sensitizer (preferably pioglitazone or a salt thereof, more preferably pioglitazone hydrochloride),
   (1b) biguanide (preferably metformin or a salt thereof, more preferably metformin hydrochloride), or
   (1c) α-glucosidase inhibitor (preferably voglibose)];
(2) an agent for pancreas protection, which comprises a blood glucose lowering drug that does not stimulate insulin secretion and 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof in combination
   [here, the blood glucose lowering drug that does not stimulate insulin secretion is preferably the following:
   (2a) insulin sensitizer (preferably pioglitazone or a salt thereof, more preferably pioglitazone hydrochloride),
   (2b) biguanide (preferably metformin or a salt thereof, more preferably metformin hydrochloride), or
   (2c) α-glucosidase inhibitor (preferably voglibose)]; and
(3) an agent for pancreas protection, which comprises a blood glucose lowering drug that does not stimulate insulin secretion and 2-[2-(3-(R)-aminopiperidin-1-yl)-5-fluoro-6-oxo-6H-pyrimidin-1-ylmethyl]benzonitrile or a salt thereof in combination
   [here, the blood glucose lowering drug that does not stimulate insulin secretion is preferably the following:
   (3a) insulin sensitizer (preferably pioglitazone or a salt thereof, more preferably pioglitazone hydrochloride),
   (3b) biguanide (preferably metformin or a salt thereof, more preferably metformin hydrochloride), or
   (3c) α-glucosidase inhibitor (preferably voglibose)].

The agent for pancreas protection of the present invention can be obtained by combining a blood glucose lowering drug that does not stimulate insulin secretion and compound (I) or compound (II) as active ingredients. These active ingredients may be formed into preparations separately or simultaneously together with a pharmacologically acceptable carrier.
Here, as the pharmacologically acceptable carrier, various organic or inorganic carrier materials conventionally used as materials for pharmaceutical preparations are used. They are blended as excipient, lubricant, binder or disintegrant for solid preparations; and solvent, solubilizing agent, suspending agent, isotonicity agent, buffer, soothing agent and the like for liquid preparations. Where necessary, an additive for pharmaceutical preparations such as preservative, antioxidant, colorant, sweetening agent and the like can be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate, magnesium aluminate metasilicate and the like.
Preferable examples of the lubricant include magnesium stearate, calcium stearate, talc, colloidal silica and the like.
Preferable examples of the binder include pregelatinized starch, saccharose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropyl methylcellulose, polyvinylpyrrolidone and the like.
Preferable examples of the disintegrant include lactose, sucrose, starch, carboxymethylcellulose, calcium carboxymethylcellulose, sodium croscarmellose, sodium carboxymethyl starch, light anhydrous silicic acid, low-substituted hydroxypropylcellulose and the like.
Preferable examples of the solvent include water for injection, physiological saline, Ringer's solution, alcohol, propylene glycol, polyethylene glycol, sesame oil, corn oil, olive oil, cottonseed oil and the like.
Preferable examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.
Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil; and the like.
Preferable examples of the isotonicity agent include sodium chloride, glycerol, D-mannitol, D-sorbitol, glucose and the like.
Preferable examples of the buffer include phosphate buffer, acetate buffer, carbonate buffer, citrate buffer and the like.
Preferable examples of the soothing agent include benzyl alcohol and the like.
Preferable examples of the preservative include p-oxybenzoates, chlorobutanol, benzyl alcohol, phenethyl alcohol, dehydroacetic acid, sorbic acid and the like.
Preferable examples of the antioxidant include sulfite, ascorbate and the like.
Preferable examples of the colorant include aqueous edible tar pigments (e.g., foodcolors such as Food Color Red Nos. 2 and 3, Food Color Yellow Nos. 4 and 5, Food Color Blue Nos. 1 and 2 etc.), water insoluble lake pigments (e.g., aluminum salt of the aforementioned aqueous edible tar pigment), natural pigments (e.g., beta carotene, chlorophyll, red iron oxide and yellow ferric oxide) and the like.
Preferable examples of the sweetening agent include saccharin sodium, dipotassium glycyrrhizinate, aspartame, stevia and the like.

Examples of the dosage form of the agent for pancreas protection of the present invention include oral preparations such as tablet (including sugar-coated tablet, film-coated tablet, sublingual tablet, orally disintegrating tablet), capsule (including soft capsule, microcapsule), granule, powder, troche, syrup, emulsion, suspension, film (e.g., mouth cavity disintegrating film) and the like; or parenteral preparations such as injections (e.g., subcutaneous injections, intravenous injections, intramuscular injections and intraperitoneal injections), external agents (e.g., preparations for nasal administration, transdermal preparations and ointments), suppositories (e.g., rectal suppositories and vaginal suppositories), pellets, drops, eye drops, pulmonary preparations (inhalations) and the like. In addition, these preparations may be sustained-release preparations (e.g., sustained-release microcapsule), such as an immediate release preparation or a sustained-release preparation. Of these preparations, oral preparations superior in the convenience or compliance are preferable.
The agent for pancreas protection of the present invention can be produced according to a method conventionally used in the field of pharmaceutical preparation, such as the method described in Japan Pharmacopoeia and the like.
While the content of the active ingredient (blood glucose lowering drug that does not stimulate insulin secretion, and/or compound (I) or compound (II)) in the agent for pancreas protection of the present invention varies depending on the kind of the active ingredient, the size of the preparation and the like, it is, for example, 1-90 wt%, preferably 5-80 wt%.

In the agent for pancreas protection of the present invention, the mixing ratio of a blood glucose lowering drug that does not stimulate insulin secretion, and compound (I) or compound (II) can be appropriately determined according to the subject of administration, administration route, target disease, dosage form, combination of pharmaceutical agents and the like. For example, compound (I) or compound (II) is generally used in an amount of about 0.005-200 parts by weight, preferably about 0.01-100 parts by weight, relative to 1 part by weight of the blood glucose lowering drug that does not stimulate insulin secretion.
The administration mode of the agent for pancreas protection of the present invention is not particularly limited, and a blood glucose lowering drug that does not stimulate insulin secretion, and compound (I) or compound (II) only need to be combined on administration. Such administration mode includes, for example,
(1) administration of a single preparation obtained by simultaneously formulating a blood glucose lowering drug that does not stimulate insulin secretion, and compound (I) or compound (II), (2) simultaneous administration of two kinds of preparations obtained by separately formulating a blood glucose lowering drug that does not stimulate insulin secretion, and compound (I) or compound (II), by the same administration route, (3) administration of two kinds of preparations obtained by separately formulating a blood glucose lowering drug that does not stimulate insulin secretion, and compound (I) or compound (II), at different times by the same administration route, (4) simultaneous administration of two kinds of preparations obtained by separately formulating a blood glucose lowering drug that does not stimulate insulin secretion, and compound (I) or compound (II), by different administration routes, (5) administration of two kinds of preparations obtained by separately formulating a blood glucose lowering drug that does not stimulate insulin secretion, and compound (I) or compound (II), at staggered times by different administration routes (e.g., administration in the order of a blood glucose lowering drug that does not stimulate insulin secretion and then compound (I) or compound (II), or in the reverse order) and the like.

The agent for pancreas protection of the present invention can be safely administered orally or parenterally to mammals (e.g., humans, mice, rats, rabbits, dogs, cats, bovines, horses, swines, monkeys).
Particularly, the agent for pancreas protection of the present invention is effective for type 2 diabetes patients, and further, effective for type 2 diabetes patients with pancreatic fatigue. Here, pancreatic fatigue can be defined using HOMA-β as an index. For example, HOMA-β is a value calculated by [fasting blood insulin level µU/mL)x 360]/[fasting blood glucose level (mg/dL)-63](%), and when the HOMA-β is not more than 50%, preferably not more than 30%, the pancreas is exhausted.
The dose of the agent for pancreas protection of the present invention can be similar to the dose of a blood glucose lowering drug that does not stimulate insulin secretion, or compound (I) or compound (II), which are the active ingredients, and can be appropriately determined according to the subject of administration, administration route, target disease, dosage form, combination of pharmaceutical agents and the like.
The dose of the blood glucose lowering drug that does not stimulate insulin secretion, and compound (I) or compound (II) can be appropriately determined based on their clinical doses.
The dose of the blood glucose lowering drug that does not stimulate insulin secretion is, for example, generally 0.01-5000 mg/day, preferably 0.1-3000 mg/day, for one adult patient (body weight 60 kg). This amount can be administered in 2 or 3 portions a day.
When an insulin sensitizer is used as a blood glucose lowering drug that does not stimulate insulin secretion, the dose of the insulin sensitizer is generally 0.1-100 mg/day, preferably 1-60 mg/day for one adult patient (body weight 60 kg).
Particularly, when the insulin sensitizer is pioglitazone hydrochloride, the effective amount of pioglitazone hydrochloride is generally 7.5-60 mg/day, preferably 15-45 mg/day, for one adult patient (body weight 60 kg), as pioglitazone.
When the insulin sensitizer is rosiglitazone maleate, the effective amount of rosiglitazone maleate is generally 1-12 mg/day, preferably 2-8 mg/day, for one adult patient (body weight 60 kg).
When a biguanide (preferably metformin hydrochloride) is used as a blood glucose lowering drug that does not stimulate insulin secretion, the dose of the biguanides (preferably metformin hydrochloride) is generally 125-2550 mg/day, preferably 250-2550 mg/day for one adult patient (body weight 60 kg).
When α-glucosidase inhibitor is used as a blood glucose lowering drug that does not stimulate insulin secretion, the dose of α-glucosidase inhibitor is generally 0.01 - 1000 mg/day, preferably 0.1 - 500 mg/day, for one adult patient (body weight 60 kg). Particularly, when α-glucosidase inhibitor is voglibose, the effective amount of voglibose is generally 0.1 - 2 mg/day, preferably 0.1 - 1mg/day, for one adult patient (body weight 60 kg).
In addition, when the α-glucosidase inhibitor is acarbose, the effective amount of acarbose is generally 10 - 500 mg/day, preferably 20 - 200 mg/day, for one adult patient (body weight 60 kg).
The dose of compound (I) or compound (II) is, for example, generally 0.1 - 1000 mg/day, preferably 1 - 500 mg/day, for one adult patient (body weight 60 kg). This amount can also be administered in 2 or 3 portions a day.

The agent for pancreas protection of the present invention has a superior pancreas protective action. Examples of the pancreas protective action include pancreatic insulin content-increasing action, pancreatic fatigue-preventing or treating action, pancreas (β cell) function-ameliorating effect, pancreas (β cell) regenerating action, pancreas (β cell) regeneration promoting action, glucotoxicity suppressing action, lipotoxicity suppressing action, glucolipotoxicity suppressing action, oxidative stress suppressing action, endoplasmic reticulum stress suppressing action, pancreatic β cell apoptosis suppressive action, insulin secretion enhancing action and the like. Here, insulin secretion enhancing action can be evaluated by calculating the ratio of plasma insulin value and plasma glucose value of a subject for administration, where an increase in the "plasma insulin value/plasma glucose value" means enhancement of insulin secretion. The pancreas protective action is preferably pancreatic insulin content-increasing action.
The agent for pancreas protection of the present invention shows a synergistic pancreas protective action. Here, the "synergistic pancreas protective action" means a pancreas protective action superior to the sum of "pancreas protective action of a blood glucose lowering drug that does not stimulate insulin secretion alone" and "pancreas protective action of compound (I) or compound (II) alone".
In the present specification, the "pancreatic insulin content" means an insulin content of the pancreas. The "pancreatic insulin content" can be obtained by measuring insulin extracted by a method known *per se* from a pancreatic tissue of a test animal according to a method known per se. The measurement method of insulin may be any as long as insulin can be measured. Specifically, a radioimmunoassay or enzyme immunoassay using one kind of anti-insulin antibody; an enzyme immunoassay using two kinds of anti-insulin antibodies having different epitopes and the like can be used.
In addition, the "pancreatic insulin content" can also be evaluated with pancreatic insulin mRNA or the amount of pancreatic β cells as an index.
Here, the pancreatic insulin mRNA and the amount of pancreatic β cells can be measured by a method known *per se.* For example, a method by histological staining using an anti-insulin antibody is generally used for the measurement of the amount of pancreatic β cells. In addition, *in situ* hybridization that detects insulin mRNA, a method including labeling with an endogenous or exogenous substance that highly specifically binds to a protein selectively expressed in pancreatic β cells, administering the labeled substance to a test animal, and thereafter measuring the labeling activity and the like may be used. The aforementioned endogenous or exogenous substance can be labeled, for example, with a radioisotope, a luminescence substance (low-molecular-weight compound or protein such as luciferase, GFP and the like), a fluorescent substance and the like.
Moreover, the "pancreatic insulin content" can also be evaluated by a known method used for the assumption of the amount of remaining pancreatic β cells. As such method, for example, a method including a glucagon tolerance test and measurement of activated insulin or C-peptide in the blood and the like can be mentioned. Alternatively, a glucose loading test may be conducted instead of the glucagon tolerance test and then the activated insulin or C-peptide in the blood may be measured. Furthermore, the activated insulin or C-peptide in the blood may be measured without a glucagon tolerance test.

The agent for pancreas protection of the present invention is useful for the prophylaxis or treatment of diabetes [e.g., type 1 diabetes, type 2 diabetes, type 1.5 (LADA (Latent Autoimmune Diabetes in Adults)), gestational diabetes mellitus, insulin secretion-deficient diabetes, obese diabetes, IGT (Impaired Glucose Tolerance), IFG (Impaired Fasting Glucose), IFG (Impaired Fasting Glycaemia)], diabetic complications [e.g., neuropathy, nephropathy, retinopathy, cataract, macroangiopathy, arteriosclerosis, osteopenia, hyperosmolar diabetic coma, infectious diseases (e.g., respiratory infection, urinary tract infection, gastrointestinal infection, dermal soft tissue infections and inferior limb infection), diabetic gangrene, xerostomia, hypacusis, cerebrovascular disorder and peripheral blood circulation disorder], and the like. In addition, the agent for pancreas protection of the present invention can suppress progression of diabetes into diabetic complications (particularly, diabetic neuropathy, diabetic nephropathy, diabetic retinopathy, arteriosclerosis).
In a mammal having a higher blood glucose level than the normal level, since hyperglycemia itself decreases the pancreatic insulin content, the agent for pancreas protection of the present invention can be used for normalization of the blood glucose level in a mammal having a higher blood glucose level than the normal level. Moreover, the agent for pancreas protection of the present invention is useful for a mammal with low pancreas (β cell) function and insulin secretion-deficient condition, from among the mammals having a higher blood glucose level than the normal level.
Furthermore, the agent for pancreas protection of the present invention can increase the amount of pancreatic insulin mRNA. The agent for pancreas protection of the present invention suppresses pancreatic fatigue caused by glucotoxicity due to diabetes, lipotoxicity, glucolipotoxicity, oxidant stress, endoplasmic reticulum stress and the like, and can maintain the glucose-dependent insulin secretory ability, which is an important function of pancreatic β cells. In addition, the agent for pancreas protection of the present invention can suppress pancreatic β cell death due to diabetes, and promote regeneration or replication of pancreatic β cells. Moreover, the agent for pancreas protection of the present invention can promote production of giant pancreatic β cells.

The agent for pancreas protection of the present invention can be used in combination with other pharmaceutical agent (hereinafter to be abbreviated as a combination drug), as long as a blood glucose lowering drug that does not stimulate insulin secretion, and compound (I) or compound (II), which are active ingredients, are not adversely affected.
Here, as the "combination drug", therapeutic agents for diabetes, therapeutic agents for diabetic complications, therapeutic agents for hyperlipidemia, antihypertensive agents, antiobesity agents, diuretic agents, antithrombotic agents and the like can be mentioned.
The timing of administration of the agent for pancreas protection of the present invention and a combination drug is not limited. They may be simultaneously administered to an administration subject or administered in a staggered manner. Moreover, the agent for pancreas protection of the present invention and a combination drug may be administered as two kinds of preparations each containing an active ingredient, or may be administered as a single preparation containing both active ingredients.
The dose of the combination drug can be appropriately determined based on the dose clinically employed. The proportion of the agent for pancreas protection of the present invention and combination drug can be appropriately determined depending on the administration subject, administration route, target disease, condition, combination and the like. When, for example, the administration subject is human, a combination drug is used in an amount of 0.01-100 parts by weight per 1 part by weight of the active ingredient of the agent for pancreas protection of the present invention.

As the aforementioned therapeutic agent for diabetes, for example, insulin preparations (e.g., animal insulin preparations extracted from the pancreas of bovine and swine; human insulin preparations genetically synthesized using *Escherichia coli* or yeast; zinc insulin; protamine zinc insulin; fragment or derivative of insulin (e.g., INS-1), GLP-1 receptor agonists [e.g., GLP-1, NN-2211, AC-2993 (exendin-4), BIM-51077, Aib(8,35)hGLP-1(7,37)NH₂], amylin agonists (e.g., pramlintide), phosphotyrosine phosphatase inhibitors (e.g., sodium vanadate), β3 agonists (e.g., AJ-9677), gluconeogenesis inhibitors (e.g., glycogen phosphorylase inhibitor, glucose-6-phosphatase inhibitor, glucagon antagonist), SGLT (sodium-glucose cotransporter) inhibitors (e.g., T-1095), adiponectin or agonist thereof, IKK inhibitors (e.g., AS-2868), leptin resistance improving drugs, glucokinase activators (e.g., Ro-28-1675), JNK inhibitor, GSK3β inhibitor and the like can be mentioned.
Examples of the therapeutic agent for diabetic complications include aldose reductase inhibitors (e.g., Tolrestat, Epalrestat, Zenarestat, Zopolrestat, Minalrestat, Fidarestat, Ranirestat etc.), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole etc.)), neuranagenesis stimulators (e.g., Y-128), PKC inhibitors (e.g., ruboxistaurin mesylate), AGE inhibitors (e.g., ALT946, pimagedine, N-phenacylthiazolium bromide (ALT766), EXO-226), reactive oxygen scavengers (e.g., thioctic acid), cerebral vasodilators (e.g., tiapride, mexiletine) and apoptosis signal regulating kinase-1 (ASK-1) inhibitors.

Examples of the therapeutic agent for hyperlipidemia include HMG-CoA reductase inhibitors (e.g., pravastatin, simvastatin, lovastatin, atorvastatin, fluvastatin, itavastatin, rosuvastatin, pitavastatin and salts thereof (e.g., sodium salt, calcium salt), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, plant sterols (e.g., soysterol, γ-oryzanol) and the like.
Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121), Clonidine and the like.

Examples of the antiobesity agent include antiobestic agents acting on the central nervous system (e.g., Dexfenfluramine, fenfluramine, phentermine, Sibutramine, amfepramone, dexamphetamine, mazindol, phenylpropanolamine, clobenzorex; MCH receptor antagonists (e.g., SB-568849; SNAP-7941; compounds encompassed in WO01/82925 and WO01/87834); neuropeptide Y antagonists (e.g., CP-422935); cannabinoid receptor antagonists (e.g., SR-141716, SR-147778); ghrelin antagonist; pancreatic lipase inhibitors (e.g., orlistat, ATL-962), β3 agonists (e.g., AJ-9677), peptidic anorexiants (e.g., leptin, CNTF (Ciliary Neurotropic Factor)), cholecystokinin agonists (e.g., lintitript, FPL-15849) and the like.
Examples of the diuretic agent include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.
Examples of the antithrombotic agent include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

The present invention further relates to "an agent for pancreas protection containing compound (I) or compound (II)".
The "agent for pancreas protection containing compound (I) or compound (II)" can be produced by using compound (I) or compound (II) in the same manner as in the method of the above-mentioned agent for pancreas protection of the present invention. The "agent for pancreas protection containing compound (I) or compound (II)" has superior pancreas protective action, preferably pancreatic insulin content increasing action, and can be used for the prophylaxis or treatment of diabetes and the like, like the aforementioned agent for pancreas protection of the present invention.

Compound (I) and compound (II) are also useful as therapeutic agents for diabetes with secondary sulfonylurea failure, and afford superior insulin secretion effect and blood glucose decreasing effect even for diabetic patients for whom sulfonylurea compounds and fact-acting insulin secretagogues fail to provide insulin secretion effect, due to which sufficient blood glucose lowering effect cannot be achieved.
Here, as the sulfonylurea compound, a compound having a sulfonylurea skeleton, a derivative thereof, for example, tolbutamide, glibenclamide, gliclazide, chlorpropamide, tolazamide, acetohexamide, glyclopyramide, glimepiride, glipizide, glybuzole and the like can be mentioned.
As the fast-acting insulin secretagogue, a compound free of a sulfonylurea skeleton but promotes insulin secretion from pancreatic β cells, like a sulfonylurea compound, for example, glinide compounds such as repaglinide, senaglinide, nateglinide, mitiglinide, a calcium salt hydrate thereof and the like, and the like can be mentioned.
Furthermore, the "agent for pancreas protection containing compound (I) or compound (II)" can be used in combination with the aforementioned combination drug.

The present invention is explained in more detail by the following Experimental Examples. These do not limit the present invention and the present invention can be modified within the range that does not deviate from the scope of the invention.

### [Experimental Example 1]

Using BKS.Cg-+ Lepr^{db}/+ Lepr^{db}/Jcl mouse (hereinafter to be abbreviated as db/db mouse) (6-week-old, male, Clea Japan, Inc.), which is a diabetes model with lowered pancreatic insulin content (type 2 diabetes model with pancreatic fatigue), and BKS.Cg-m +/+ Lepr^{db}/Jcl mouse (hereinafter to be abbreviated as db/+m mouse) (6-week-old, male, Clea Japan, Inc.), which is a normal model, a pancreatic insulin content increasing effect, afforded by a combination of a blood glucose lowering drug that does not stimulate insulin secretion, and compound A, was studied.
First, the db/db mice (16 mice) were divided into 2 Groups A and B (each 8 mice), to Group A (control group) was given a powder diet (trade name: CE-2, Clea Japan, Inc., hereinafter the same), to Group B was given a powder diet containing pioglitazone hydrochloride (0.0075(w/w)% as pioglitazone) and compound A (0.03(w/w)% of compound A in a free form) [dose of pioglitazone and free form compound A for Group B was 14.1±0.8 (mean±SD) mg/kg body weight/day and 56.5±3.1 (mean+SD) mg/kg body weight/day, respectively], each for 25 consecutive days.
In addition, a powder diet was given to db/+m mice (4 mice) for 25 days, and the mice were used as Group C (normal group).
No significant difference was observed in the food intake of the mice in the above-mentioned Groups A - C.
In the above-mentioned Groups A - C, pancreas was isolated from the mice at day 27 from the start of the powder diet administration, and the pancreatic insulin content was measured.
The pancreatic insulin content was measured as follows.
First, the mouse pancreas was homogenated in its 10 times weight of 74% ethanol (containing 0.15 mM hydrochloric acid). The obtained homogenate solution was left standing overnight under the shading conditions at 4°C, and centrifuged at 15000 rpm for 5 min. The obtained supernatant was appropriately diluted with PBS(-) (phosphate-buffered saline) containing 0.1% BSA (bovine serum albumin). The insulin content of the diluted solution was measured by radioimmunoassay (trade name: RAT INSULIN RIA KIT, manufactured by RINCO Research, U.S.A.), and the insulin content per pancreatic tissue weight was calculated.
The results are shown in Table 1. The values in the Table indicate mean (n=8 in Groups A and B; n=4 in Group C)±standard deviation.

**[Table 1]**

| Pancreatic insulin content (ng/mg tissue) | | |
|---|---|---|
| Group | | Pancreatic insulin content |
| Group A | (control) | 44.0 ± 17 |
| Group B | (pioglitazone + compound A) | 200.1 ± 138 |
| Group C | (normal group) | 198.9 ± 16 |

As shown in Table 1, the combination of the blood glucose-lowering drug that does not stimulate insulin secretion and compound A afforded a superior pancreatic insulin content-increasing effect.

### [Experimental Example 2]

The pancreatic insulin content-increasing effect afforded by a combination of a blood glucose lowering drug that does not stimulate insulin secretion and compound A was further studied.
The db/db mice (24; male, 6-week-old, CLEA Japan, Inc.) were divided into 4 Groups A - D (each 6 mice), to Group A (control group) was given a powder diet (trade name: CE-2, Clea Japan, Inc., hereinafter the same), to group B was given a powder diet containing voglibose (0.001 (w/w)%) [dose of voglibose for Group B was 1.8±0.2 (mean±SD) mg/kg body weight/day], to Group C was given a powder diet containing compound A (0.03 (w/w)% of compound A in a free form) [dose of free form compound A for Group C was 72.8±4.4 (mean±SD) mg/kg body weight/day], and to Group D was given a powder diet containing voglibose (0.001 (w/w)%) and compound A (0.03 (w/w)% of compound A in a free form) [dose of voglibose and free form compound A for Group D was 1.8±0.3 (mean±SD) mg/kg body weight/day and 53.8±9.2 (mean±SD) mg/kg body weight/day, respectively], each for 27 consecutive days.
No significant difference was observed in the food intake of the mice in the above-mentioned groups A to D.
In the above-mentioned Groups A - D, pancreas was isolated from the mice at day 29 from the start of the powder feed administration, and the pancreatic insulin content was measured.
The insulin content per pancreatic tissue weight was calculated in the same manner as in Experimental Example 1. The results are shown in Table 2. The values in the Table indicate mean (n=6)±standard deviation.

**[Table 2]**

| Pancreatic insulin content (ng/mg tissue) | | |
|---|---|---|
| Group | | Pancreatic insulin content |
| Group A | (control) | 58.0 ± 23.1 |
| Group B | (voglibose) | 198.1 ± 169.8 |
| Group C | (compound A) | 92.0 ± 37.0 |
| Group D | (voglibose + compound A) | 492.0 ± 189.5 |

As shown in Table 2, the combination of the blood glucose-lowering drug that does not stimulate insulin secretion and compound A afforded a superior pancreatic insulin content-increasing effect.

### [Experimental Example 3]

The pancreatic insulin content-increasing effect afforded by a combination of a blood glucose lowering drug that does not stimulate insulin secretion and compound B was studied.
The db/db mice (28; male, 6-week-old, CLEA Japan, Inc.) were divided into 4 Groups A - D (each 7 mice), to Group A (control group) was given a powder diet (trade name: CE-2, Clea Japan, Inc., hereinafter the same), to group B was given a powder diet containing pioglitazone hydrochloride (0.0075(w/w)% as pioglitazone) [dose of pioglitazone for Group B was 17.7±1.7 (mean+SD) mg/kg body weight/day], to Group C was given a powder diet containing compound B (0.03 (w/w)% of compound B in a free form) [dose of free form compound B for Group C was 74.7±6.3 (mean±SD) mg/kg body weight/day], and to Group D was given a powder diet containing pioglitazone hydrochloride (0.0075 (w/w)%) and compound B (0.03 (w/w)% of compound B in a free form) [dose of pioglitazone and free form compound B for Group D was 15.8±1.2 (mean±SD) mg/kg body weight/day and 63.1±4.9 (mean±SD) mg/kg body weight/day, respectively], each for 26 consecutive days.
No significant difference was observed in the food intake of the mice in the above-mentioned groups A to D.
In the above-mentioned Groups A - D, pancreas was isolated from the mice at day 28 from the start of the powder feed administration, and the pancreatic insulin content was measured.
The insulin content per pancreatic tissue weight was calculated in the same manner as in Experimental Example 1. The results are shown in Table 3. The values in the Table indicate mean (n=7)±standard deviation.

**[Table 3]**

| Pancreatic insulin content (ng/mg tissue) | | |
|---|---|---|
| Group | | Pancreatic insulin content |
| Group A | (control) | 33.7 ± 10.8 |
| Group B | (pioglitazone) | 74.8 ± 21.5 |
| Group C | (compound B) | 58.1 ± 17.0 |
| Group D | (pioglitazone + compound B) | 173.1 ± 67.1 |

As shown in Table 3, the combination of the blood glucose-lowering drug that does not stimulate insulin secretion and compound B afforded a superior pancreatic insulin content-increasing effect.

### [Experimental Example 4]

The pancreatic insulin content-increasing effect afforded by a combination of a blood glucose lowering drug that does not stimulate insulin secretion and compound C was studied.
The db/db mice (28; male, 6-week-old, CLEA Japan, Inc.) were divided into 4 Groups A - D (each 7 mice), to Group A (control group) was given a powder diet (trade name: CE-2, Clea Japan, Inc., hereinafter the same), to group B was given a powder diet containing pioglitazone hydrochloride (0.0075(w/w)% as pioglitazone) [dose of pioglitazone for Group B was 17.7±1.7 (mean±SD) mg/kg body weight/day], to Group C was given a powder diet containing compound C (0.1 (w/w)% of compound C in a free form) [dose of free form compound C for Group C was 245.8±17.3 (mean±SD) mg/kg body weight/day], and to Group D was given a powder diet containing pioglitazone hydrochloride (0.0075 (w/w)% as pioglitazone) and compound C (0.1 (w/w)% of compound C in a free form) [dose of pioglitazone and free form compound C for Group D was 17.3±1.3 (mean±SD) mg/kg body weight/day and 230.3±17.5 (mean±SD) mg/kg body weight/day, respectively], each for 26 consecutive days.
No significant difference was observed in the feed intake of the mice in the above-mentioned groups A to D.
In the above-mentioned Groups A - D, pancreas was isolated from the mice at day 28 from the start of the powder feed administration, and the pancreatic insulin content was measured.
The insulin content per pancreatic tissue weight was calculated in the same manner as in Experimental Example 1. The results are shown in Table 4. The values in the Table indicate mean (n=7)±standard deviation.

**[Table 4]**

| Pancreatic insulin content (ng/mg tissue) | | |
|---|---|---|
| Group | | Pancreatic insulin content |
| Group A | (control) | 33.7 ± 10.8 |
| Group B | (pioglitazone) | 74.8 ± 21.5 |
| Group C | (compound C) | 57.2 ± 17.8 |
| Group D | (pioglitazone + compound C) | 116.1 ± 47.8 |

As shown in Table 4, the combination of the blood glucose-lowering drug that does not stimulate insulin secretion and compound C afforded a superior pancreatic insulin content-increasing effect.

### Industrial Applicability

The agent for pancreas protection of the present invention provides a superior pancreatic insulin content increasing effect and is useful for the treatment of diabetes and the like.
This application is based on patent application Nos. 2005-379407 (filing date: December 28, 2005) and 2006-061722 (filing date: March 7, 2006) filed in Japan, the contents of which are all hereby incorporated.

## Claims

1. An agent for pancreas protection, which comprises a blood glucose lowering drug that does not stimulate insulin secretion, and compound (I) or compound (II) in combination, wherein the compound (I) is a compound represented by the formula (I): 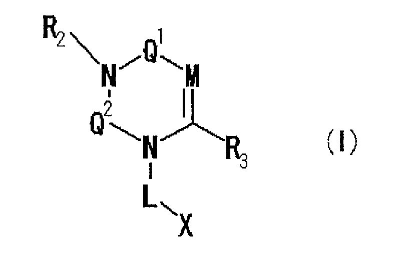 wherein M is N or CR₄;
Q¹ and Q² are each independently selected from the group consisting of CO, CS, SO, SO₂ and C=NR₁;
R₂ is a hydrogen atom, or a group selected from the group consisting of (C₁₋₁₀)alkyl, (C₃₋₁₂)cycloalkyl, (C₃₋₁₂)cycloalkyl(C₁₋₅)alkyl, hetero(C₃₋₁₂)cycloalkyl(C₁₋₅)alkyl, hetero(C₃₋₁₂)cycloalkyl, (C₆₋₁₀)aryl(C₁₋₁₀)alkyl, heteroaryl(C₁₋₅)alkyl, (C₉₋₁₂)bicycloaryl, hetero(C₄₋₁₂)bicycloaryl, hetero(C₄₋₁₂)bicycloaryl(C₁₋₅)alkyl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, amino, (C₆₋₁₀)aryl, heteroaryl, hydroxy, (C₁₋₁₀)alkoxy, (C₆₋₁₀)aryloxy, heteroaryloxy, carbonyl group, imino group, sulfonyl group and sulfinyl group (each of these can be substituted or unsubstituted);
R₃ is a group selected from the group consisting of perhalo(C₁₋₁₀)alkyl, amino, (C₁₋₁₀)alkyl, (C₃₋₁₂)cycloalkyl, hetero(C₃₋₁₂)cycloalkyl, (C₆₋₁₀)aryl(C₁₋₁₀)alkyl, heteroaryl(C₁₋₅)alkyl, (C₆₋₁₀)aryl, heteroaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, hydroxy, (C₁₋₁₀)alkoxy, (C₆₋₁₀)aryloxy, heteroaryloxy, carbonyl group, imino group, sulfonyl group and sulfinyl group (each of these can be substituted or unsubstituted), and a substituted or unsubstituted 3-, 4-, 5-, 6- or 7-membered ring;
R₄ is a hydrogen atom, or a group selected from the group consisting of halo, perhalo(C₁₋₁₀)alkyl, amino, cyano, thio, (C₁₋₁₀)alkyl, (C₃₋₁₂)cycloalkyl, hetero(C₃₋₁₂)cycloalkyl, (C₆₋₁₀)aryl(C₁₋₁₀)alkyl, heteroaryl(C₁₋₅)alkyl, (C₆₋₁₀)aryl, heteroaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, hydroxy, (C₁₋₁₀)alkoxy, (C₆₋₁₀)aryloxy, heteroaryloxy, a carbonyl group, an imino group, a sulfonyl group and a sulfinyl group (each of these can be substituted or unsubstituted);
R₁ is a hydrogen atom, or a group selected from the group consisting of (C₁₋₁₀)alkyl, (C₃₋₁₂)cycloalkyl, hetero(C₃₋₁₂)cycloalkyl, (C₆₋₁₀)aryl(C₁₋₁₀)alkyl, heteroaryl(C₁₋₅)alkyl, (C₉₋₁₂)bicycloaryl, and hetero(C₄₋₁₂)bicycloaryl (each of these can be substituted or unsubstituted);
L is a linker providing a gap corresponding to 1, 2 or 3 atoms between X and the ring L is bonded to (the atom of the linker is selected from the group consisting of a carbon atom, an oxygen atom, a nitrogen atom and a sulfur atom); and
X is a group selected from the group consisting of (C₁₋₁₀)alkyl, (C₃₋₁₂) cycloalkyl, hetero (C₃₋₁₂) cycloalkyl, (C₆₋₁₀)aryl(C₁₋₁₀)alkyl, heteroaryl (C₁₋₅)alkyl, (C₉₋₁₂)bicycloaryl, hetero(C₄₋₁₂)bicycloaryl, carbonyl (C₁₋₃) alkyl, thiocarbonyl(C₁₋₃)alkyl, sulfonyl (C₁₋₃)alkyl, sulfinyl (C₁₋₃) alkyl, imino (C₁₋₃)alkyl, amino, (C₆₋₁₀)aryl, heteroaryl, hydroxy, (C₁₋₁₀)alkoxy, (C₆₋₁₀)aryloxy, heteroaryloxy, (C₂₋₆)alkenyl, (C₂₋₆)alkynyl, a carbonyl group, cyano, an imino group, a sulfonyl group and a sulfinyl group (each of these can be substituted or unsubstituted), or a salt thereof, and
the compound (II) is a compound represented by the formula (II) : 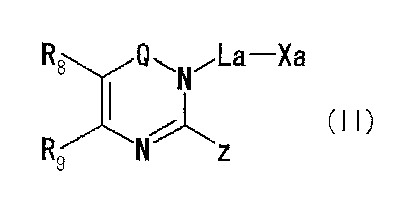 wherein Q is selected from the group consisting of CO, SO, SO₂ and C=NR₇;
Z is a group selected from the group consisting of halo, perhalo(C₁₋₁₀)alkyl, amino, cyano, thio, (C₁₋₁₀)alkyl, cycloalkyl, heterocycloalkyl, aryl, heteroaryl, carbonyl(C₁₋₃)alkyl, thiocarbonyl(C₁₋₃)alkyl, sulfonyl (C₁₋₃)alkyl, sulfinyl(C₁₋₃)alkyl, imino(C₁₋₃)alkyl, hydroxy, alkoxy, aryloxy, heteroaryloxy, alkenyl, alkynyl, a carbonyl group, an imino group, a sulfonyl group and a sulfinyl group (each of these can be substituted or unsubstituted), and a substituted or unsubstituted 4-, 5-, 6- or 7-membered ring;
R₈ and R₉ are each independently a hydrogen atom, or a group selected from the group consisting of halo, perhalo(C₁₋₁₀)alkyl, amino, cyano, nitro, thio, (C₁₋₁₀)alkyl, (C₃₋₁₂)cycloalkyl, hetero(C₃₋₁₂)cycloalkyl, aryl(C₁₋₁₀)alkyl, heteroaryl(C₁₋₅)alkyl, (C₉₋₁₂)bicycloaryl, hetero(C₈₋₁₂)bicycloaryl, carbonyl(C₁₋₃)alkyl, thiocarbonyl(C₁₋₃))alkyl, sulfonyl(C₁₋₃)alkyl, sulfinyl(C₁₋₃)alkyl, imino(C₁₋₃)alkyl, aryl, heteroaryl, hydroxy, alkoxy, aryloxy, heteroaryloxy, alkenyl, alkynyl, a carbonyl group, an imino group, a sulfonyl group and a sulfinyl group (each of these can be substituted or unsubstituted);
R₇ is a hydrogen atom, or a group selected from the group consisting of (C₁₋₁₀)alkyl, cycloalkyl, heterocycloalkyl, aryl, heteroaryl, arylalkyl, heteroarylalkyl, bicycloaryl and heterobicycloaryl (each of these can be substituted or unsubstituted);
La is a linker providing a gap corresponding to 0 to 6 atoms between Xa and the ring La is bonded to;
Xa is a group selected from the group consisting of (C₁₋₁₀)alkyl, (C₃₋₁₂)cycloalkyl, hetero(C₃₋₁₂)cycloalkyl, aryl(C₁₋₁₀)alkyl, heteroaryl(C₁₋₅)alkyl, (C₉₋₁₂)bicycloaryl, hetero(C₄₋₁₂)bicycloaryl, carbonyl(C₁₋₃)alkyl, thiocarbonyl(C₁₋₃)alkyl, sulfonyl(C₁₋₃)alkyl, sulfinyl(C₁₋₃)alkyl, imino(C₁₋₃)alkyl, amino, aryl, heteroaryl, hydroxy, alkoxy, aryloxy, heteroaryloxy, alkenyl, alkynyl, a carbonyl group, cyano, an imino group, a sulfonyl group and a sulfinyl group (each of these can be substituted or unsubstituted), or a salt thereof

2. The agent of claim 1, wherein the blood glucose lowering drug that does not stimulate insulin secretion is an insulin sensitizer.

3. The agent of claim 2, wherein the insulin sensitizer is pioglitazone or a salt thereof.

4. The agent of claim 1, wherein the blood glucose lowering drug that does not stimulate insulin secretion is a biguanide.

5. The agent of claim 4, wherein the biguanide is metformin or a salt thereof.

6. The agent of claim 1, wherein the blood glucose lowering drug that does not stimulate insulin secretion is an α-glucosidase inhibitor.

7. The agent of claim 6, wherein the α-glucosidase inhibitor is voglibose.

8. An agent for pancreas protection, which comprises a blood glucose lowering drug that does not stimulate insulin secretion and 2-[[6-[(3R)-3-amino-1-piperidinyl]-3,4-dihydro-3-methyl-2,4-dioxo-1(2H)-pyrimidinyl]methyl]benzonitrile or a salt thereof in combination.

9. An agent for pancreas protection, which comprises a blood glucose lowering drug that does not stimulate insulin secretion and 2-[[6-[(3R)-3-amino-1-piperidinyl]-3,4-dihydro-3-methyl-2,4-dioxo-1(2H)-pyrimidinyl]methyl]-4-fluorobenzonitrile or a salt thereof in combination.

10. An agent for pancreas protection, which comprises a blood glucose lowering drug that does not stimulate insulin secretion and 2-[2-(3-(R)-aminopiperidin-1-y1)-5-fluoro-6-oxo-6H-pyrimidin-1-ylmethyl]benzonitrile or a salt thereof in combination.

11. A method of pancreas protection of a mammal, which comprises administering a blood glucose lowering drug that does not stimulate insulin secretion and compound (I) or compound (II) to the mammal, wherein the compound (I) is a compound represented by the formula (I): 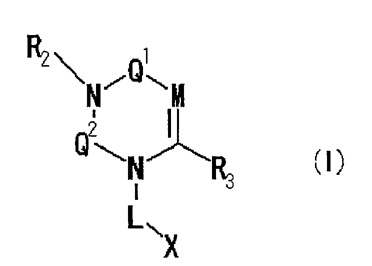 wherein each symbol is as defined in claim 1, or a salt thereof, and
the compound (II) is a compound represented by the formula (II): 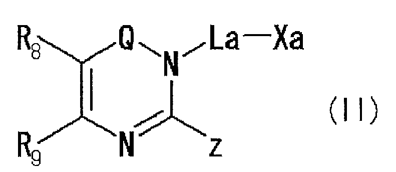 wherein each symbol is as defined in claim 1, or a salt thereof.

12. Use of a blood glucose lowering drug that does not stimulate insulin secretion and compound (I) or compound (II) for the production of an agent for pancreas protection, wherein the compound (I) is a compound represented by the formula (I): 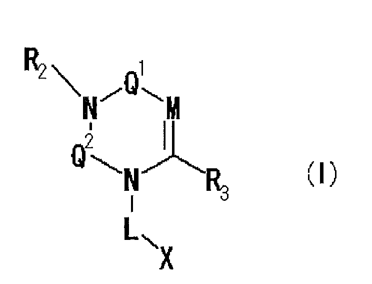 wherein each symbol is as defined in claim 1, or a salt thereof, and
the compound (II) is a compound represented by the formula (II) : 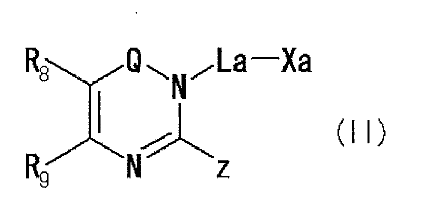 ) wherein each symbol is as defined in claim 1, or a salt thereof.
